# EUROPEAN PATENT APPLICATION

(11) **EP 3 974 526 A1**
(43) Date of publication of application: **30.03.2022**
(21) Application number: 20382849.6
(22) Date of filing: 25.09.2020
(51) Int. Cl.: C12N 9/24, C12N 15/63, C12P 19/14

(54) **XYLANASE ENZYME WITH EXTREME THERMOSTABILITY AND ALKALINE STABILITY**

(71) Applicant: Consejo Superior de Investigaciones Científicas (CSIC), 28006 Madrid (ES)
(72) Inventor: POLAINA MOLINA, Julio, 46980 Paterna (Valencia) (ES); TALENS PERALES, David, 46980 Paterna (Valencia) (ES); SÁNCHEZ TORRES, Paloma, 46980 Paterna (Valencia) (ES)
(74) Representative: Pons

(57) **Abstract**

The present invention relates to the use of an isolated polypeptide characterized in that it comprises at least 70% identity with the amino acid sequence SEQ ID NO: 1 or the nucleotide encoding thereof for the hydrolysis of xylan, wherein the hydrolysis reaction occurs at a temperature of at least 65 °C and at a pH of at least 8.0. This polypeptide shows extreme thermostability and alkaline stability which allow its use in different process such as paper making, fodder production, fermenting composting, improving dough handling, extraction of coffee, extraction of plant oils, extraction of starch, agriculture silage, clarification of juices and degumming of plant fiber sources.

## Description

The present invention relates to the uses of a xylanase enzyme comprising an amino acid sequence having at least 70% identity with the amino acid sequence SEQ ID NO: 1, and showing extreme thermostability and alkaline stability, which allows its use in different process such as paper making, fodder production, fermenting composting, improving dough handling, extraction of coffee, extraction of plant oils, extraction of starch, agriculture silage, clarification of juices and degumming of plant fiber sources. Thus, the present invention relates to the technical field of enzymes and compositions thereof.

### BACKGROUND ART

Xylan is a group of hemicelluloses that represents the third most abundant biopolymer on Earth. It is found in plants, the secondary cell walls of dicots and all the cell walls of grasses.

Xylanase is main enzyme that degrades hemicellulose among sugar hydrolysis enzymes, and is widely applied in various fields such as food, animal feeds, textiles and paper making. For example, xylanase may be used to treat fodder to degrade anti-nutrient factors in the feed, thereby promoting the uptaking of nutrient and the growth of animals. Xylanase can be added to dough to improve the mechanical strength of the dough as well as its appearance and storability. Furthermore, xylanase is used in the paper making industry in the paper and pulp processing, where xylanases extract the lignin from the pulp, leading to a reduction in the chemicals required to bleach the pulp and produce the whiteness of the paper.

Due to is importance in so many industrial processes, especially in paper processing, xylanases enzymes have been the subject of several works with the objective of obtaining xylanases with improved yield, improved stability at high temperatures or both. Due to this several xylanases have been isolated from thermophiles, filamentous fungi and Archae. For example, the patent document US5902581 discloses a xylanase derived from *Acidothermus cellulolyticus*, the xylanase exhibiting enzymatic activity at 60 to 80°C and a pH of 3.6 to 4.2. The document US6083733 discloses a xylanase derived from an anaerobic thermostable bacterium isolated from a New Zealand hot spring, and this xylanase also exhibits enzymatic activity at 60 to 80°C at a pH of 9.0 or above. The document WO2016073610 describes a composition used in the process of pulp bleaching which contains a xylanase enzyme obtained from *Dictyoglomus thermophilum* with activity at 60 to 95 °C and at a pH of 5 to 10. The document EP 2990482 discloses a xylanase belonging to the family GH10 which exhibits activity at a temperature of 85 °C and a pH of 6.0.

Despite the previous mentioned documents, these disclose xylanases which are thermostable and acidophilic, have low high temperatures of activity or have been demonstrated to work in a specific process (e.g. pulp bleaching). Therefore, xylanase enzymes which present high thermostability together with high alkaline stability in the degradation of hemicellulose are required to be used in the several processes previously exposed.

### SUMMARY OF THE INVENTION

The present invention discloses a novel xylanase enzyme obtained from *Pseudothermotoga thermarum* bacteria which, surprisingly, presents thermostability at high temperatures and alkaline stability at high pH values, which allows the use of said xylanase for the degradation of cellulose and hemicellulose, activity that finds applications in a wide variety of industrial processes which depend or greatly benefit from the degradation of these polymers.

The inventors isolated this xylanase by bioinformatics screening of the protein sequences belonging to Glycosyl Hydrolases Families GH10 and GH11 included in the CAZY Database. Starting from the proteins selected, the corresponding nucleotide sequences encoding said proteins were edited, synthetized and subjected to tests for assessing its hydrolytic capacities at different temperatures and values of pH using as substrate oat spelt xylan (see Example 1). The inventors noted that the nucleotide sequence SEQ ID NO: 11 encoding to the xylanase comprising the amino acid sequence SEQ ID NO: 1 was able to keep the 80% of hydrolytic activity at 90°C and pH 10.5.

Therefore, a first aspect of the invention relates to the use of
- an isolated polypeptide comprising an amino acid sequence having an identity of at least 70% with the sequence SEQ ID NO: 1, or a fragment thereof having xylanase activity (Here onwards "polypeptide of the invention");
- a nucleotide sequence that codifies for said polypeptide (Here onwards "nucleotide sequence of the invention");
- a vector comprising said nucleotide sequence (Here onwards "vector of the invention");
- a host cell comprising said polypeptide, said nucleotide sequence or said vector (Here onwards, "cell of the invention");
- or a composition comprising said polypeptide, said nucleotide sequence, said vector, said host cell or any combination of thereof (Here onwards, "composition of the invention"),
for the hydrolysis of xylan, wherein the hydrolysis reaction occurs at a temperature of at least 65 °C and at a pH of at least 8.0.

The term "isolated" means a substance in a form or environment which does not occur in nature. Non-limiting examples of isolated substances include (1) any non-naturally occurring substance, (2) any substance including, but not limited to, any enzyme, variant, nucleic acid, protein, peptide or cofactor, that is at least partially removed from one or more or all of the naturally occurring constituents with which it is associated in nature; (3) any substance modified by the hand of man relative to that substance found in nature; or (4) any substance modified by increasing the amount of the substance relative to other components with which it is naturally associated (e.g., multiple copies of a gene encoding the substance; use of a stronger promoter than the promoter naturally associated with the gene encoding the substance). An isolated substance may be present in a fermentation broth sample. Thus, the term "isolated polypeptide" as used herein refers to any amino acid molecule comprising two or more amino acids joined to each other by peptide bonds or modified peptide bonds, i.e. peptide isosteres, which has been removed from its natural environment as explained above. Polypeptides may contain amino acids other than the 20 gene-encoded amino acids. Polypeptides may include amino acid sequences modified either by natural processes, such as post-translational processing, or by chemical modification techniques which are well known in the art. Modifications can occur anywhere in a polypeptide, including the polypeptide backbone, the amino acid side-chains and the amino or carboxyl termini. The same type of modification may be present in the same or varying degrees at several sites in a given polypeptide. Also, a given polypeptide may contain many types of modifications.

The polypeptide of the invention comprises an amino acid sequence having an identity of at least 70% with the sequence SEQ ID NO: 1. The term "identity" or "sequence identity" as used herein refers to the degree of similarity between two nucleotide or amino acid sequences obtained by aligning the two sequences, i.e. the proportion of identical nucleotide or amino acids between two compared nucleotide sequences or polypeptides/proteins along their full-length sequence. Depending on the number of common residues between the aligned sequences, a different degree of identity, expressed as a percentage, will be obtained. The degree of identity between two amino acids or nucleotides sequences may be determined by conventional methods, for example, by standard sequence alignment algorithms known in the state of the art, such as BLAST [Altschul S.F. et al. Basic local alignment search tool. J Mol Biol. 1990 Oct 5; 215(3): 403-10]. The BLAST programmes, for example, BLASTN, BLASTX, and T BLASTX, BLASTP and TBLASTN, are in the public domain at *The National Center for Biotechonology Information* (NCBI) website. The skilled person in the art understands that mutations in the nucleotide sequence of genes that lead to conservative amino acid substitutions at non-critical positions for the functionality of the protein are evolutionarily neutral mutations which do not affect its global structure or its functionality.

In the context of the present invention, it is considered that that peptides, polypeptides or proteins with an identity of at least 70% with SEQ ID NO: 1 are functionally equivalent variants of the SEQ ID NO: 1 and maintain the same properties as the sequence to which they refer, i.e. they show xylanase activity at a temperature of at least 65 °C and at a pH of at least 8.0. An assay for assessing if a given peptide, polypeptide or protein is a functionally equivalent variant of the sequence SEQ ID NO: 1 can be found in the examples of the present description. In a preferred embodiment, the polypeptide of the invention has an identity of at least 80, 85, 90, 91, 92, 93, 94, 95, 96, 97, 98 or 99% with amino acid sequence SEQ ID NO: 1. In a more preferred embodiment, the polypeptide of the invention comprises, or consists of, SEQ ID NO: 1. The polypeptide comprising the amino acid sequence SEQ ID NO: 1 was obtained by bioinformatics analysis and found that it corresponded to the bacteria *Pseudothermotoga thermarum.* Said polypeptide shows xylanase activity, i.e., it is a xylanase enzyme (EC. 3.2.1.8) that, as mentioned above, hydrolyzes or degrades the linear polysaccharide xylan (CAS number: 9014-63-5) into xylose (CAS number: 85-86-6).

SEQ ID NO: 1
GPQPMSSQIPSLKDVFLQDFKIGVALPVRVFSNSMDVELITKHFNSMTAENEMKPESILRRDASGKIY
YDFTVADRYIEFAQKHGMVVRGHTLVWHSQTPEWFFKDEKGNLLSREAMIERMREYIHTVVGRYRGKV
YAWDVVNEAVDENQPDGLRRSLWYQVIGPDYIELAFKFAHEADPDALLFYNDYNEFFPKKRDIIFKLV
KEMREKGVPIHGIGMQQHLTLADNVGWIDIAIQKFKTISGIQIHITELDVSVYKSRSPSIIYQTPPLE
VLKEQAEFYRKLFEIYRKHTDVITNVTFWGLKDDYSWLRFFFGRRNDWPLLFDENYQPKPAFWSVIES
VSK

The term "functionally equivalent variant" refers to a protein (or polypeptide) comprising one or more alterations, such as substitutions, insertions, deletions and/or truncations of one or more specific amino acid residues at one or more specific positions in the protein, and having the same function that the original protein. Thus, variants of the proteins encoded by the biomarkers of the invention may be (i) one in which one or more of the amino acid residues are substituted with a conserved or non-conserved amino acid residue (preferably a conserved amino acid residue) and such substituted amino acid residue may or may not be one encoded by the genetic code, (ii) one in which there are one or more modified amino acid residues, e.g., residues that are modified by the attachment of substituent groups, (iii) one in which the protein is an alternative splice variant of the proteins of the present invention and/or (iv) fragments of the proteins. The fragments include proteins generated via proteolytic cleavage (including multi-site proteolysis) of an original sequence. Variants are deemed to be within the scope of those skilled in the art from the teaching herein. The term "fragment" means a protein or a domain thereof having one or more (e.g., several) amino acids absent from the amino and/or carboxyl terminus of the amino acid sequence, wherein the fragment has the same activity than the whole protein.

The term "xylanase" means endo-acting glycoside hydrolase (E.C.3.2.1.8) that catalyses the endohydrolysis of 1,4-beta-D-xylosidic linkages in xylans. Xylanase enzymes are part of a wider group of enzymes called Glycoside hydrolases, known for hydrolyzing the glycosidic bond between two or more carbohydrates. A classification system for glycoside hydrolases, based on sequence similarity of the glycoside hydrolase domain, has led to the definition of >100 different families. The polypeptide of the invention is part of Glycoside hydrolase family 10 (GH10). GH10 enzymes can further be grouped depending on their domain architecture (DA). GH10 polypeptides with more complex DA, contain other domains like carbohydrate-binding module 4-9 (CBM4-9), CBM2 and Ricin-B-lectin domains. CBM4-9 binds to amorphous cellulose and other soluble oligosaccharides, but not to crystalline cellulose. CBM2 binds to cellulose but also to other polymers such as chitin. Ricin B lectin domains are also related with sugar recognition.

The polypeptide of the invention may comprise a signal peptide linked to the N-terminal end useful to direct the polypeptide into the cell's secretory pathway when it is produced. Thus, in a preferred embodiment, the polypeptide of the invention further comprises a signal sequence at the N-terminal end. Any signal peptide can be used in the context of the present invention. Nevertheless, in a more preferred embodiment of the polypeptide of the invention, the signal peptide comprises an amino acid sequence having an identity of at least 70, 75, 80, 85, 90, 91, 92, 93, 94, 95, 96, 97, 98 or 99% with the sequence SEQ ID NO: 2. In a still more preferred embodiment, the signal peptide comprises, or consists of, the sequence SEQ ID NO: 2.

SEQ ID NO: 2
MKKLIFAITLLLIVFTVIIKG
In a more preferred embodiment of the use of the invention, the polynucleotide of the invention comprises, or consists of, the amino acid sequence SEQ ID NO: 3.

SEQ ID NO: 3
MKKLIFAITLLLIVFTVIIKGGPQPMSSQIPSLKDVFLQDFKIGVALPVRVFSNSMDVELITKHFNSM
TAENEMKPESILRRDASGKIYYDFTVADRYIEFAQKHGMVVRGHTLVWHSQTPEWFFKDEKGNLLSRE
AMIERMREYIHTVVGRYRGKVYAWDVVNEAVDENQPDGLRRSLWYQVIGPDYIELAFKFAHEADPDAL
LFYNDYNEFFPKKRDIIFKLVKEMREKGVPIHGIGMQQHLTLADNVGWIDIAIQKFKTISGIQIHITE
LDVSVYKSRSPSIIYQTPPLEVLKEQAEFYRKLFEIYRKHTDVITNVTFWGLKDDYSWLRFFFGRRND
WPLLFDENYQPKPAFWSVIESVSK

The polypeptide of the invention is characterized by comprising the single GH10 domain. The inventors created variants of the polypeptide of the invention where different domains were added to the polypeptide of the invention to try and improve the enzymatic characteristics of the polypeptide of the invention. Therefore, in a preferred embodiment, the polypeptide of the invention further comprises the CBM2 domain or the CBM9 domain. In a more preferred embodiment, the CBM2 domain comprises, or consists of, the sequence SEQ ID NO: 4.

SEQ ID NO: 4

TTTPVPVSGSLEVKVNDWGSGAEYDVTLNLDGQYDWTVKVKLAPGATVGSFWSANKQEGNGYVIFTPV

SWNKGPTATFGFIVNGPQGDKVEEITLEINGQVIEA

In another preferred embodiment, the CBM9 domain comprises, or consists of, the sequence SEQ ID NO: 5.

SEQ ID NO: 5

GIMVATAKYGTPVIDGEIDEIWNTTEEIETKAVAMGSLDKNATAKVRVLWDENYLYVLAIVKDPVLNK

DNSNPWEQDSVEIFIDENNHKTGYYEDDDAQFRVNYMNEQTFGTGGSPARFKTAVKLIEGGYIVEAAI

KWKTIKPTPNTVIGFNIQVNDANEKGQRVGIISWSDPTNNSWRDPSKFGNLRLIK

This variant of the polypeptide of the invention showed an increase in stability of the hydrolysis of xylan at a large range of pH and temperature.

The polypeptide of the invention may be a fusion polypeptide or cleavable fusion polypeptide in which another polypeptide is fused at the N-terminus or the C-terminus of the polypeptide of the present disclosure. A fusion polypeptide is produced by fusing a polynucleotide encoding another polypeptide to a polynucleotide of the present disclosure. Techniques for producing fusion polypeptides are known in the art, and include ligating the coding sequences encoding the polypeptides so that they are in frame and that expression of the fusion polypeptide is under control of the same promoter(s) and terminator. Fusion polypeptides may also contain "linker polypeptides", i.e., small peptide sequence between 2 - 20 amino acids which connects two polypeptides domain together. Therefore, in a preferred embodiment of the polypeptide of the invention further comprises a linker polypeptide that connects the polypeptide of the invention to the CBM2 domain or the CBM9 domain. In a more preferred embodiment, the linker polypeptide comprises or consists of SEQ ID NO: 6 (VDGA). In yet another preferred embodiment the polypeptide of the invention comprises or consists of a sequence that is selected from the list consisting of: SEQ ID NO: 7 y SEQ ID NO: 8.

The polypeptide of the invention, as well as all its particular embodiments above-disclosed, can be codified by a nucleotide sequence, which can be equally used for the hydrolysis of xylan according to the first aspect of the invention. The term "nucleotide" refers to the basic building blocks of nucleic acids comprising or consisting of a nucleobase bound, ribose or deoxyribose and at least one phosphate group. The basic building blocks are cytosine (C), guanine (G), adenine (A), thymine (T), and uracil (U). The term "nucleotide sequence" as used herein refers to an oligonucleotide sequence or polynucleotide sequence, and variants, homologues, fragments and derivatives thereof (such as portions thereof). The nucleotide sequence may be DNA or RNA of genomic or synthetic or recombinant origin which may be double-stranded or single-stranded whether representing the sense or antisense strand. Preferably, the term "nucleotide sequence" means DNA. More preferably, the term "nucleotide sequence" means DNA prepared by use of recombinant DNA techniques (i.e. recombinant DNA).

Modification of a polynucleotide encoding a polypeptide of the invention may be by introduction of nucleotide substitutions that do not result in a change in the amino acid sequence of the polypeptide, but which correspond to the codon usage of the host organism intended for production of the enzyme. Thus, in a preferred embodiment of the use of the invention, the nucleotide sequence encoding to the polypeptide of the invention is optimized for its expression in *E. coli.* In a more preferred embodiment, the nucleotide sequence encoding to the polypeptide of the invention comprises, or consists of, the nucleotide sequence SEQ ID NO: 9.

SEQ ID NO: 9
GGACCACAGC CCATGTCGTC ACAGATACCA TCCCTTAAGG ATGTTTTTTT
GCAGGATTTC AAAATAGGAG TAGCTTTACC AGTTAGAGTT TTCAGCAATT
CGATGGATGT GGAGTTAATT ACGAAGCACT TTAACAGTAT GACGGCTGAG
AACGAGATGA AACCAGAGAG TATATTGAGA AGGGATGCGA GTGGGAAGAT
TTACTATGAT TTTACGGTTG CAGACAGGTA CATAGAGTTT GCGCAGAAAC
ATGGGATGGT TGTTAGGGGT CATACACTTG TTTGGCACAG TCAGACGCCG

GAGTGGTTTT TCAAGGATGA GAAGGGCAAT TTATTGAGCA GGGAAGCGAT
GATAGAGAGG ATGCGGGAGT ACATACACAC AGTTGTAGGC AGGTATAGAG
GCAAGGTGTA TGCGTGGGAT GTGGTGAACG AGGCGGTGGA TGAGAATCAG
CCTGATGGTT TGAGGAGGAG TTTGTGGTAT CAGGTTATAG GGCCGGATTA
CATAGAGCTT GCCTTCAAAT TTGCTCACGA AGCAGATCCA GACGCCTTAC
TCTTCTACAA CGATTACAAC GAATTTTTCC CCAAAAAGCG AGACATCATC
TTCAAATTGG TAAAAGAAAT GAGAGAAAAG GGTGTACCGA TTCATGGTAT
TGGCATGCAG CAACATTTAA CTCTTGCAGA TAACGTAGGT TGGATTGATA
TTGCTATTCA AAAATTCAAG ACCATTTCAG GTATACAAAT TCACATAACT
GAGCTTGATG TTAGTGTTTA CAAATCACGC AGTCCATCTA TTATCTATCA
AACGCCACCC TTGGAGGTGC TCAAGGAGCA AGCGGAATTT TACCGCAAGT
TGTTCGAAAT CTATAGGAAA CACACTGACG TCATAACCAA CGTTACCTTC
TGGGGTTTGA AAGATGACTA TTCGTGGCTA AGGTTTTTCT TTGGTCGTAG
AAACGATTGG CCGCTTTTAT TTGACGAAAA TTATCAACCC AAACCAGCTT
TTTGGAGTGT AATAGAATCA GTTTCCAAGT AA

The nucleotide sequence of the invention may comprise a signal peptide coding region that encodes a signal peptide linked to the N-terminus of a variant and directs the variant into the cell's secretory pathway. The 5'-end of the coding sequence of the polynucleotide may inherently contain a signal peptide coding sequence naturally linked in translation reading frame with the segment of the coding sequence that encodes the polypeptide. Alternatively, the 5'-end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the variant. However, any signal peptide coding sequence that directs the expressed variant into the secretory pathway of a host cell may be used.

Effective signal peptide coding sequences for bacterial host cells include, without limiting to, the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (nprT, nprS, nprM), and *Bacillus subtilis* prsA.

Effective signal peptide coding sequences for filamentous fungal host cells include, without limiting to, the signal peptide coding sequences obtained from the genes for *Aspergillus niger* neutral amylase, *Aspergillus niger* glucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells include, without limiting to, those obtained from the genes for *Saccharomyces cerevisiae* alpha-factor and *Saccharomyces cerevisiae* invertase.

In a preferred embodiment, the nucleotide sequence encoding the signal peptide comprises, or consists of, the sequence SEQ ID NO: 10.

SEQ ID NO: 10

ATG AAA AAG CTG ATT TTC GCC ATC ACA TTG TTG CTT ATA GTC TTT ACT
GTG ATA ATC AAA GGA

The nucleotide of the invention may further comprise linker nucleotides that links nucleotide sequence regions to each other, wherein said regions may be functional domain regions. The linker nucleotides allow for functional domains to be linked without the function of the domains be altered or modified. In a preferred embodiment the nucleotide of the invention further comprises a linker nucleotide. In a preferred embodiment the linker nucleotide comprises, or consists of, the sequence SEQ ID NO: 11.
SEQ ID NO: 11
GTCGACGGCG CA

In a more preferred embodiment of the use of the invention, the nucleotide sequence of the invention comprises, or consists of, the sequence selected from a list consisting of: SEQ ID NO: 9, SEQ ID NO: 12, SEQ ID NO: 13, SEQ ID NO: 14, SEQ ID NO: 15, SEQ ID NO: 16, SEQ ID NO: 17 and SEQ ID NO: 18.

The nucleotide sequence of the invention be manipulated in a variety of ways to provide for expression of the polypeptide, such as the optimization of codons for the expression of the polypeptide in a specific host cell. Manipulation of the polynucleotide prior to its insertion into a vector may be desirable or necessary depending on the expression vector. The techniques for modifying polynucleotides utilizing recombinant DNA methods are well known in the art.

The polypeptide of the invention or a nucleotide sequence that codifies for such polypeptide can be comprised in a vector, from here onwards the vector of the invention. Said vector can be used for the hydrolysis of xylan. The term "vector" as used herein refers to a nucleic acid molecule capable of transporting one or more other nucleic acid sequence(s), like a nucleotide sequence that codifies for the polypeptide of the invention, to which it has been linked or which was introduced into said vector. One type of vector is a "plasmid", which refers to a circular double stranded DNA into which additional DNA segments may be cloned. Another type of vector is a viral vector, wherein additional DNA segments may be cloned into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) or parts thereof, are integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" or simply "expression vectors". In general, expression vectors of utility in recombinant DNA techniques are often in the form of plasmids. In the present specification, "plasmid" and "vector" may be used interchangeably, as the plasmid is the most commonly used form of vector. However, the disclosure is intended to include other forms of expression vectors, such as viral vectors (e.g., replication defective retroviruses, adenoviruses and adeno-associated viruses), which serve equivalent functions. The vector may also contain additional sequences, such as a "regulatory nucleotide sequence", a "polylinker" or "multiple cloning site" for subcloning of additional nucleic acid sequences. A "vector backbone" or "plasmid backbone" refers to a piece of DNA containing at least a plasmid origin of replication and a selectable marker which allows for selection of hosts cells containing the plasmid or vector. When a particular plasmid or vector is modified to contain non-plasmid elements (e.g. insertion of Ad sequences and/or a eukaryotic gene of interest linked to a ribosomal promoter), the plasmid sequences are referred to as the plasmid backbone. The term "promoter" as used herein refers to a nucleotide sequence required for transcription at significant levels of a second polynucleotide to which it is operably linked.

The term "regulatory nucleotide sequence" as used herein refers to a region or sequence determinants located upstream or downstream from the start of transcription and which are involved in recognition and binding of RNA polymerase and other proteins to initiate transcription. The term "regulatory nucleotide sequence" is intended to include promoters, enhancers, internal ribosomal entry sites (IRES), and other expression control elements (e.g. transcription termination signals, such as polyadenylation signals and poly-U sequences). Regulatory elements include those that direct constitutive expression of a nucleotide sequence in many types of host cell and those that direct expression of the nucleotide sequence only in certain host cells (e.g., tissue-specific regulatory sequences). In a preferred embodiment of the use of the invention the vector comprises a regulatory nucleotide sequence, preferably a promoter, operatively linked to a nucleotide sequence that codifies for the polypeptide of the invention.

The term "operatively linked" as used herein refers to that separate nucleotide sequences are functionally associated such that an event at one can precipitate a response from the other. Two or more operably linked nucleotide sequences can, in combination, comprise an independent genetic element, such as a recombinant expression cassette.

The vector of the invention can be comprised by a host cell. Said host cell, from here onwards the host cell of the invention, comprising the vector of the invention, or the polypeptide of the invention or a nucleotide sequence that codifies for the polypeptide of the invention can be used for the hydrolysis of xylan according to the first aspect of the invention.

As used herein, the term "host cell" means any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication. Because certain modifications may occur in succeeding generations due to either mutation or environmental influences, such progeny may not, in fact, be identical to the parent cell, but are still included within the scope of the term "host cell" as used herein. A host cell can be used, for example, for expression of a nucleic acid of interest, propagation of plasmid vectors and/or delivery of a sequence of interest to cells, wherein the nucleic acid of interest or plasmid vector are present episomally in these cells. The episomal vectors are not integrated into the host genome and are retained in self-replicating form in the host cell. The term episomally replicating is understood here as that the vector is not integrated to the genome of the host cell, but exists in parallel, is also replicated during the cell cycle and in the course of this the vector copies-depending on the number of the copies present before and after cell division-are distributed statistically in the resulting cells.

Examples of such cells include, without limitation, bacterial cells like *Escherichia coli* cells, yeast cells like *Saccharomyces cerevisiae, Pichia pastoris* and *Schizosaccharomyces pombe,* insect cells like *Spodoptera frugiperda* Sf21 and Sf19 cells, mammalian cells like human embryonal kidney cells, Chinese hamster ovary cells and Hella cells. In addition, host cells can also be cells obtained by integrating the nucleotide that codifies for the polypeptide of the invention, or the vector of the invention, into their genome by known methods in the art. Preferably host cells include prokaryotic and eukaryotic cells selected from any of the kingdoms of life. Several cell types are known in the art and a skilful person in the subject matter will be able to identify an ideal cell type for use in the specific application. Preferred cells include, but are not limited to, mammalian cells, avian cells, amphibian cells, plant cells, fish cells, bacterial cells and insect cells. In a more preferred embodiment, the host cell is selected from a list comprising gram-negative bacteria cells, zebrafish cells, Drosophila cells, mouse cells and non-human cells. In a preferred embodiment of the use of the invention the host cell is a bacterium, preferably the bacterium is *E. coli.*

The use of the invention also contemplates a composition, from here onwards the composition of the invention, that comprises the polypeptide of the invention, a nucleotide sequence that codifies for the polypeptide of the invention, the vector of the invention and the host cell of the invention or any combination of the aforementioned, for hydrolysis of xylan. The composition of the invention may comprise other components that are useful for the realization of the xylan hydrolysis, such as buffer solutions or liquid carriers. Lyophilized powders including compositions of this invention fall within the scope of the composition of the invention so long as the powders are intended to be reconstituted by the addition of a suitable liquid carrier prior to use. Examples of suitable liquid carriers include, but are not limited to water, distilled water, de-ionized water, saline, buffer solutions, normal isotonic saline solution, dextrose in water, and combinations thereof.

The composition of the invention may further comprise other proteins useful in degrading the cellulosic material. Thus, in another preferred embodiment, the composition of the invention further comprises at least one protein selected from the list consisting of a cellulase, a hemicellulase, a CIP, an esterase, an expansin, a laccase, a ligninolytic enzyme, a pectinase, a catalase, a peroxidase, a protease, and a swollenin. In a more preferred embodiment, the cellulase is preferably one or more (e.g., several) enzymes selected from the group consisting of an endoglucanase, a cellobiohydrolase (including, without limiting to, cellobiohydrolase I and cellobiohydrolase II), and a beta-glucosidase. In another more preferred embodiment, the hemicellulase is preferably one or more (e.g., several) enzymes selected from the group consisting of an acetylmannan esterase, an acetylxylan esterase, an arabinanase, an arabinofuranosidase, a coumaric acid esterase, a feruloyl esterase, a galactosidase, a glucuronidase, a glucuronoyl esterase, a mannanase, a mannosidase, and a xylosidase.

The term "buffer solution" or "buffer" as used herein refers to a solution containing both a weak acid and its conjugate weak base. The buffer solutions are used in compositions of this invention in order to resist pH changes. Non-limiting examples of useful buffer solutions are solutions that comprise sodium bicarbonate and sodium phosphate.

The polypeptide, nucleotide, vector, host cell and composition of the invention find their use in the hydrolysis of xylan, made possible by the xylanase action of the polypeptide of the invention. The "hydrolysis of xylan" reaction is accomplished by the xylanase cleavage of the β-1,4 linkages between the xylopyranosyl residues in xylan backbone. The polypeptide of the invention, has activity of xylanase at a temperature of at least 65, 70, 75, 80 or 85 °C and at a pH of at least 8.0, 8.5, 9.0, 9.5, 10.0, 10.5, 11.0. In a preferred embodiment of the use of the invention, the hydrolysis of xylan occurs at a temperature of at least 90 °C and a pH of 9.0.

The use of the invention can be applied to several processes where the hydrolysis of xylan is essential or useful to improve the process or the yield of the final product derived from the process.

As such, in another preferred embodiment of the use of the invention, the hydrolysis of xylan is carried out in a process selected from a list consisting of: paper making, fodder production, fermenting composting, improving dough handling, extraction of coffee, extraction of plant oils, extraction of starch, agriculture silage, clarification of juices and degumming of plant fiber sources.

In another aspect, the present invention relates to the use of a kit comprising
- the polypeptide of the invention;
- the nucleotide sequence of the invention;
- the vector of the invention;
- the host cell of the invention;
- or the composition of the invention,
as well as all their preferred embodiments as defined in the previous inventive aspects, for the hydrolysis of xylan, wherein the hydrolysis reaction occurs at a temperature of at least 65 °C and at a pH of at least 8.0.

In another aspect, the present invention relates to a process for the hydrolysis of xylan at a temperature of at least 65 °C and at a pH of at least 8.0, hereinafter "process of the invention", comprising treating the material, e.g. the cellulosic material, to be degraded with the polypeptide, the nucleotide, the vector, the host cell and/or the composition of the invention. All the particular embodiment previously described in the present description, alone or in combination, are applicable to the process of the invention.

The process may further comprise recovering the degraded or converted cellulosic material. Soluble products of degradation or conversion of the cellulosic material can be separated from insoluble cellulosic material using a method known in the art such as, for example, centrifugation, filtration, or gravity settling.

The processing of the cellulosic material according to the present invention can be accomplished using methods conventional in the art. Moreover, the processes of the present invention can be implemented using any conventional biomass processing apparatus configured to operate in accordance with the invention. conventional apparatus can include a fed-batch stirred reactor, a batch stirred reactor, a continuous flow stirred reactor with ultrafiltration, and/or a continuous plug-flow column, an attrition reactor. Additional reactor types include fluidized bed, upflow blanket, immobilized, and extruder type reactors for hydrolysis.

In practicing the processes of the present invention, any pretreatment process known in the art can be used to disrupt components of the cellulosic material. The cellulosic material can also be subjected to particle size reduction, sieving, presoaking, wetting, washing, and/or conditioning prior to pretreatment using methods known in the art. Conventional pretreatments include, but are not limited to, steam pretreatment (with or without explosion), dilute acid pretreatment, hot water pretreatment, alkaline pretreatment, lime pretreatment, wet oxidation, wet explosion, ammonia fiber explosion, organosolv pretreatment, and biological pretreatment. Additional pretreatments include ammonia percolation, ultrasound, electroporation, microwave, supercritical CO2, supercritical H2O, ozone, ionic liquid, and gamma irradiation pretreatments.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### DESCRIPTION OF THE DRAWINGS

**Figure 1****. Purified protein band by His-Tag purification (C) and soluble protein after heat shock treatment (HS) at 85 °C during 5 minutes.**
**Figure 2****. Xylanase activity of Xyn10, Xyn11 (the polypeptide of the invention) and Xyn13.** Xylanase activity was determined using 1% of oat spelt xylan and DNS assay to detect reducing sugars at different pH ranging from 5.0 to 10.5 at 65°C (A) and at different temperatures, 60 °C, 70 °C, 80 °C and 90 °C at pH 9.9 (B). Error bars indicate standard deviation of triplicates.
**Figure 3****. Xylanase activity of Xyn11 and Xyn11-CBM2** using 1% of oat spelt xylan and DNS assay to detect reducing sugars at pH 5 to 10.5 at 65 °C (A) and at 60 °C, 70 °C, 80 °C and 90 °C of temperature at pH 9.0 (B). Error bars indicate standard deviation of triplicates.
**Figure 4****. Xylanase activity of Xyn11 and Xyn11-CBM2** using 1% of oat spelt xylan and DNS assay to detect reducing sugars at different pH ranging from 5.0 to 10.5 at 90 °C.

### Example: Xylanase enzyme showing xylanase activity at a temperature of at least 65 °C and at a pH of at least 8.0.

### I - MATERIAL AND METHODS

### Synthesis and cloning of selected genes.

The selection of xylanase-encoding DNA sequences to be synthesized, produced as proteins in *E. coli* and characterized experimentally, derived from a bioinformatic analysis of putative xylanase sequences. This analysis yielded sequences Xyn10, Xyn11 (SEQ ID NO: 11), Xyn13 and Xyn14 (Genebank accession.version numbers: ACK42987.1, AEH51686.1, ACM60945.1, ADQ04077.1, ACC46361.1, respectively, as published on the 22/09/2020). Putative signal peptide sequences were detected using the Phobius Tool (Käll et al., 2007, Nucl. Acids Res., 35:W429-W434) and removed. The coding sequences were optimized for *E. coli* expression by using the Integrated DNA Technologies (IDT) Codon Optimization Tool (www.idtdna.com). Native restriction sites were eliminated and *SacI* and *SalI* restriction sites were added in 5' and 3' respectively, to facilitate the cloning in vector pQE80L (Quiagen).

Synthetic genes (codon-optimized) of sequences encoding five selected putative xylanases (Xyn10-14) were purchased from IDT. The DNA fragments (except Xyn10 and Xyn14) were digested with endonucleases *SacI* and *SalI* and cloned into pQE80L plasmid cut with the same enzymes. Xyn10 and Xyn14 could not be synthesized as a single piece and were acquired in two fragments: Xyn10-F1 and Xyn14-F1, which were cut with *SacI* and *EcoRI*, and Xyn10-F2 and Xyn14-F2 cut with *EcoRI* and *SalI* and then cloned in pQE80L. The cloned genes were amplified by PCR, using oligos AL645 (CCCGAAAAGTGCCACCTG - SEQ ID NO: 19) and AL646 (GTTCTGAGGTCATTACTGG - SEQ ID NO: 20). Fidelity of the cloned sequences was verified by sequencing. Fast Digest enzymes and T4 ligase were purchased from ThermoScientific. Hybrid enzymes Xyn11-CBM2 and Xyn11-CBM9 were constructed from plasmids Xyn6-CBM2 PQE80L and Xyn6-CBM9 (Talens-Perales et al., 2020, Biotechnol. for Biofuels, submitted), respectively, replacing the Xyn6 gene by the Xyn11 gene. The resulting plasmids were transformed into *E. coli* Rosetta (Stratagene) for protein production.

### Enzyme assays.

Selection of clones with xylanase activity was performed in 96-microtiter culture plates. *E. coli* transformants were grown in 200 microliters of LB with 100 micrograms/L ampicillin overnight at 37°C and 180 rpm. 10 microliters from each well was transferred to a new plate with fresh LB amended with ampicillin, which was further incubated until the cultures reached OD600 = 0.6. The cultures were induced with IPTG 1 mM at 16 °C overnight. The cells were collected by centrifugation, resuspended in 150 microliters of lysis buffer (phosphate buffer 50 mM pH 7.0 with 2.5 mg/mL lysozyme) and incubated 1 hour at 37°C. A volume of 20 microliters of each cell crude extract was added to 180 microliters of oat spelt xylan 1% (Sigma) in phosphate buffer 50 mM pH 6.5 and incubated at 65 °C for 30 minutes. Xylanase activity was determined by measuring reducing sugars resulting from the enzyme action. For this purpose, 100 microliters of DNS (dinitrosalicylic acid) reagent (Sigma) was added to each reaction well and the plate was incubated for 30 minutes at 85 °C. Positive clones were identified by a dark orange color. The identity of the coding sequence responsible for the activity of the xylanolytic clones was checked by DNA sequencing.

### Protein purification

Cell crude extracts were prepared from *E. coli* cultures grown at 37 °C to a cell density of 0.6 at OD600, induced by adding 1 mM IPTG and incubated either at 16 °C overnight or 37 °C for 5 hours. The cells were disrupted by sonication in buffer A (20 mM phosphate buffer, pH 7.4, 10 mM imidazole, 500 mM NaCl). Protein extracts were recovered by centrifugation at 12000 *g* during 25 minutes and subjected to nickel affinity chromatography using a 1 mililiter HisTrap FF crude column (GE Healthcare) mounted in an AKTA-Purifier equipment (GE). The protein was eluted with buffer B (20 mM phosphate buffer, pH 7.4, 500 mM imidazole, 500 mM NaCl). Eluted fractions showing xylanase activity were dialyzed against buffer C (20 mM Tris-HCI, pH 7 50 mM NaCl). The protein was analyzed by SDS-PAGE, using Blue Safe staining (Nzytech). An image of the gel was taken with a Proxima AQ-4 gel documentation system (Isogen) and the resulting amount of protein in the gel bands was quantified using FIJI software.

### Measurement of xylanase activity at different conditions of temperature and pH.

Enzyme reactions were prepared by mixing 180 microliter of substrate (1% oat spelt xylan, in Tris-HCI 50 mM buffer pH 9.0) and 20 microliter of purified enzyme (protein concentration was adjusted to the DNS assay conditions) and then incubated at 60 °C, 70 °C, 80 °C or 90 °C, for 10 minutes. The reaction was stopped by putting the tubes on ice.

Activity at the following different pH values was determined in 50 mM buffered solutions: 5.0 (acetate), 6.0 and 7.0 (phosphate), 8.0, 9.0 and 10.5 (Tris-HCI). The enzyme reactions were prepared by mixing 180 microliters of 1% oat spelt xylan (Sigma) in buffer and 20 microliters of purified protein (diluted at a concentration suitable for the DNS assay). The reactions were incubated at 65 °C during 10 minutes and then stopped on ice.

Production of reducing sugars was determined by adding 100 microliters of DNS reactive to the reaction tubes that were then boiled for 10 minutes. Next, 900 microliters of miliQ H₂O were added and the tubes were centrifuged. 300 microliters of the supernatant were transferred to 96-well plates and OD540 was measured using PowerWave HT equipment, from BioTek Instruments (Winooski, VT, USA).

### II - RESULTS

### Synthesis and characterization of enzymes

Synthetic, codon-optimized gene sequences were expressed in *E. coli* to produce the selected putative xylanases. Additionally, to evaluate the effect of carbohydrate binding modules, chimeric proteins were constructed by fusion of Xyn11 to CBM2 from *Pyrococus furiosus* (Xyn11-CBM2 - SEQ ID NO: 7) and CBM9 from *Thermotoga maritima* (Notenboom et al., 2001, Biochemistry, 40:6248-6256) (Xyn11-CBM9-SEQ ID NO: 8). Synthesis of the coding sequences of xylanase could be accomplished except for Xyn10 and Xyn14, which had to be obtained in two fragments (Xyn10-F1, Xyn14-F1 and Xyn10-F2, Xyn14-F2).

*E. coli* transformant cultures harboring plasmids with genes encoding the different xylanases were induced with IPTG. This allowed high production of His-tagged enzymes that were purified from bacterial crude cell extracts by nickel affinity chromatography. Xylanases (Xyn10, Xyn11, Xyn13, Xyn14 and hybrids Xyn11-CBM2 abd Xyn11-CBM9) were recovered with different yields after purification

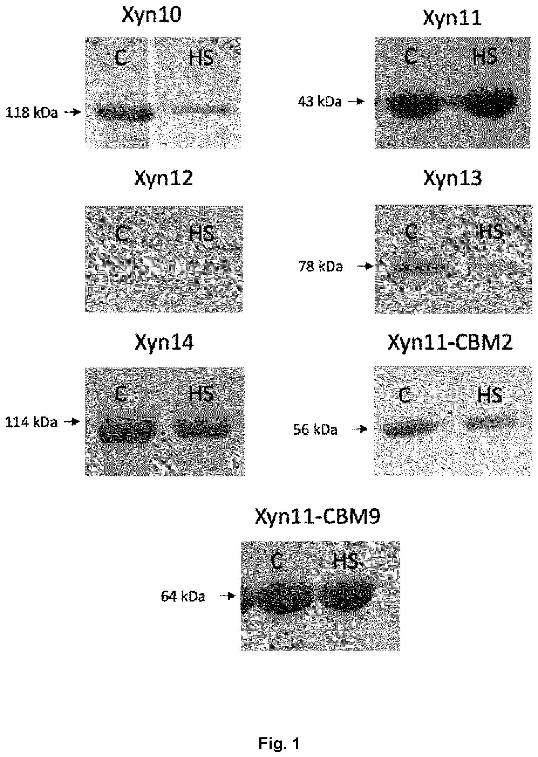

Thermal stability was analyzed by heating purified proteins at 85 °C for 5 minutes. The soluble fraction recovered after this treatment was evaluated by SDS-PAGE, in parallel to the untreated samples (Figure 1). In all cases, electrophoretic mobility was in concordance with the expected molecular mass of the xylanases, predicted by ProtParam, except for Xyn12 which signal was not detectable and was discarded for further experiments. Highest thermal stability was shown for Xyn14 and Xyn11: most protein remained soluble and enzymatically active after heat treatment. Protein from Xyn10 and Xyn13 precipitated partially after heating. Hybrid enzyme Xyn11-CBM9 showed high thermostability, while Xyn11-CBM2 was less stable.

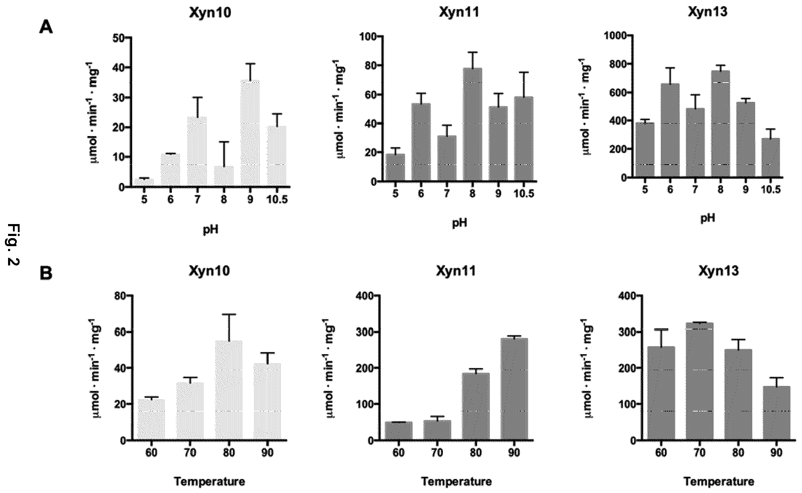

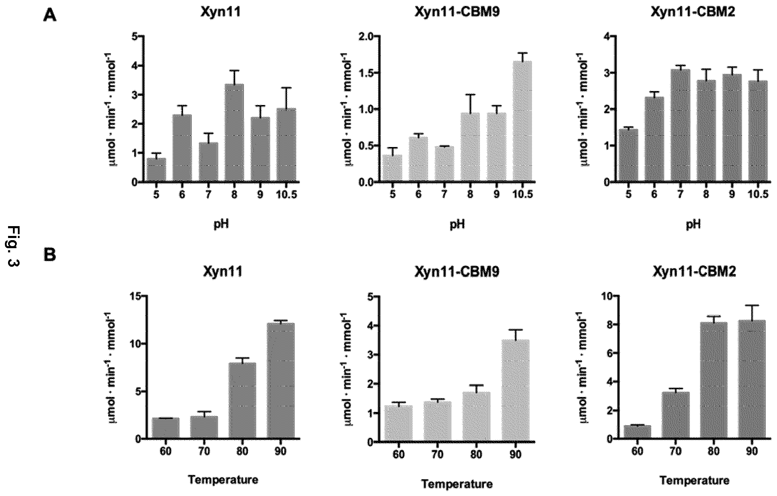

Initial semiquantitative activity evaluation exhibited that Xyn14 was clearly less active than the other enzymes and therefore were discarded. Oat spelt xylan hydrolysis by Xyn10, Xyn11, and Xyn13 was measured at different pH and temperature. Assays at different values of pH were carried out at 65 °C (Figure 2A). At 65 °C, Xyn10, 11 and 12 showed the profile of an alkalophilic enzyme, with optimal activity at pH between 8.0 and 9.0 (Figure 2A). In buffered solution at pH 9.0, selected xylanases showed a thermophilic profile, between 70-90 °C. The highest activity corresponded to Xyn11 at 90 °C (Figure 3B).

*Pseudothermotoga thermarum* produces another GH10 xylanase with a more complex DA, that contains three CBM4-9 in N-terminal position and two CBM9 domains at C-terminal position. The catalytic domain of this enzyme is very similar to that of Xyn11 and they debranch from the same node in the phylogenetic tree. This enzyme, named as Xyn10A, was characterized and was optimally active at 95°C and pH 7.0 (Shi et al., 2013, Biotechnology for Biofuels, 6, 26, 1 - 9). Related thermostable xylanases have also been described. Some of these, from *Bacillus,* show optimal temperatures around 70 °C and pH ranging from 6.5 to 9.0. Only *Bacillus halodurans* TSEV xylanase approaches Xyn11 properties, with optimal values of pH and temperature of 9.0 and 80 °C, respectively (Wang et al., 2019, Biotechnology for biofuels, 12, 48, 1 - 13). Xylanases from *Geobacillus, Caldicellusiruptor* and *Thermotoga,* show optimal performance at high temperature, remarkably *Thermotoga naphthophila* RKU-10 and *Thermotoga. petrophila* RKU-1 (95 °C) or *Thermotoga maritima* MSB8 (95 °C), but they have neutral or acidic optimal pH: 6.0, 6.0 and 5.0, respectively (ul Haq et al., 2012, Mol Biol Rep.;39(7):7251-7261).

Overall, pH and temperature profiles of Xyn11 yielded the best results for a thermophilic, alkalophilic xylanase, showing high activity at pH 10.5 and 90 °C. Taking into consideration these results, hybrid enzymes Xyn11-CBM2 and Xyn11-CBM9 were analyzed. Addition of the CBM9 domain modifies the pH optimum profile of the enzyme, decreasing the activity at some pH value but increasing it at pH10.5 (Figure 3A). Fusion of Xyn11 to CBM2 changed the profile of activity at different pH values, assayed at 65 °C (Figure 3A). Xyn11-CBM2 displays higher activity at pH 7.0, 8.0 and 9.0 compared with Xyn11.

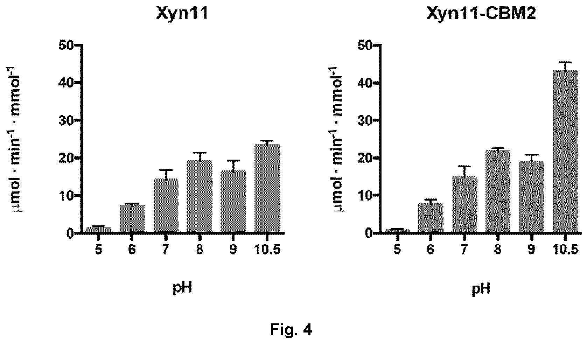

Xyn11 and its hybrids were assayed under alkaline conditions (pH 9.0) and different temperatures. Xyn11-CBM9 exhibited a temperature profile similar to Xyn11 but with a sharp decrease of activity (Figure 3B). Xyn11-CBM2 shows slightly higher activity at 70 and 80 °C, but lower activity (ca. 80%) at 90 °C (Figure 3B). However, when the activity was assayed at 90 °C and different pH values the hybrid showed a maximum of activity (ca. 40 micromole of reducing sugar. min⁻¹ · milimole of enzyme⁻¹, at pH 10.5) (Figure 4).

## Claims

1. Use of
- an isolated polypeptide comprising an amino acid sequence having an identity of at least 70% with the sequence SEQ ID NO: 1, or a fragment thereof having xylanase activity;
- a nucleotide sequence that codifies for said polypeptide;
- a vector comprising said nucleotide sequence;
- a host cell comprising said polypeptide, said nucleotide sequence or said vector;
- or a composition comprising said polypeptide, said nucleotide sequence, said vector, said host cell or any combination of thereof, for the hydrolysis of xylan, wherein the hydrolysis reaction occurs at a temperature of at least 65 °C and at a pH of at least 8.0.

2. The use according to claim 1, wherein the hydrolysis of xylan occurs at a temperature of 90 °C and a pH of 9.0.

3. The use according to claims 1 or 2, wherein the pH is of 10, preferably 10.5.

4. The use according to any one of claims 1 to 3, wherein the host cell is a bacterium, preferably *E*. *coli.*

5. The use according to any one of claims 1 to 4, wherein the polypeptide comprises an identity of at least 80, 85, 90, 95, 96, 97, 98, 99% with amino acid sequence SEQ ID NO: 1, preferably, the polypeptide comprises, or consists of, an identity of 100% with SEQ ID NO: 1.

6. The use according to any one of the claims from 1 to 5, wherein the polypeptide further comprises a signal peptide and/or a linker peptide, preferably, the signal peptide comprises the sequence SEQ ID NO: 2 and the linker peptide comprises the sequence SEQ ID NO: 6.

7. The use according to any one of the claims from 1 to 5, wherein the polypeptide further comprises carbohydrate-binding module (CBM) domain, preferably, the CMB domain comprises the amino acid sequence SEQ ID NO: 4 or SEQ ID NO: 5.

8. The use according to any one of the claims from 1 to 6, wherein the nucleotide sequence that codifies the polypeptide comprises the sequence selected from the list consisting of: SEQ ID NO: 9, SEQ ID NO: 12, SEQ ID NO: 13, SEQ ID NO: 14, SEQ ID NO: 15, SEQ ID NO: 16, SEQ ID NO: 17 and SEQ ID NO: 18.

9. The use according to any one of the claims 1 to 8, wherein the hydrolysis of xylan is carried out in a process selected from a list consisting of: paper making, fodder production, fermenting composting, improving dough handling, extraction of coffee, extraction of plant oils, extraction of starch, agriculture silage, clarification of juices and degumming of plant fiber sources.

10. Use of a kit comprising
- an isolated polypeptide comprising an amino acid sequence having an identity of at least 70% with the sequence SEQ ID NO: 1, or a fragment thereof having xylanase activity;
- a nucleotide sequence that codifies for said polypeptide;
- a vector comprising said nucleotide sequence;
- a host cell comprising said polypeptide, said nucleotide sequence or said vector;
- or a composition comprising said polypeptide, said nucleotide sequence, said vector, said host cell or any combination of thereof
- for the hydrolysis of xylan, wherein the hydrolysis reaction occurs at a temperature of at least 65 °C and at a pH of at least 8.0.

11. The use according to claim 9, wherein the hydrolysis of xylan occurs at a temperature of 90 °C and a pH of 9.0.

12. The use according to claims 9 or 10, wherein the pH is of 10, preferably 10.5.

13. The use according to any one of claims 9 to 12, wherein the polypeptide comprises an identity of at least 80, 85, 90, 95, 96, 97, 98, 99% with amino acid sequence SEQ ID NO: 1, preferably, the polypeptide comprises and identity of 100% with SEQ ID NO: 1.

14. The use according to any one of the claims from 9 to 14, wherein the nucleotide sequence that codifies the polypeptide comprises the sequence selected from the list consisting of: SEQ ID NO: 9, SEQ ID NO: 12, SEQ ID NO: 13 and SEQ ID NO: 14.

15. The use according to any one of the claims 9 to 14, wherein the hydrolysis of xylan is carried out in a process selected from a list consisting of: paper making, fodder production, fermenting composting, improving dough handling, extraction of coffee, extraction of plant oils, extraction of starch, agriculture silage, clarification of juices and degumming of plant fiber sources.


SEQUENCE LISTING

<110> CONSEJO SUPERIOR DE INVESTIGACIONES CIENTÍFICAS (CSIC)

<120> Xylanase enzyme with extreme thermostability and alkaline stability

<130> EP1641.1555

<160> 20

<170> BiSSAP 1.3.6

<210> 1
<211> 343
<212> PRT
<213> Pseudothermotoga thermarum

<400> 1
Gly Pro Gln Pro Met Ser Ser Gln Ile Pro Ser Leu Lys Asp Val Phe
1               5                   10                  15
Leu Gln Asp Phe Lys Ile Gly Val Ala Leu Pro Val Arg Val Phe Ser
            20                  25                  30
Asn Ser Met Asp Val Glu Leu Ile Thr Lys His Phe Asn Ser Met Thr
        35                  40                  45
Ala Glu Asn Glu Met Lys Pro Glu Ser Ile Leu Arg Arg Asp Ala Ser
    50                  55                  60
Gly Lys Ile Tyr Tyr Asp Phe Thr Val Ala Asp Arg Tyr Ile Glu Phe
65                  70                  75                  80
Ala Gln Lys His Gly Met Val Val Arg Gly His Thr Leu Val Trp His
                85                  90                  95
Ser Gln Thr Pro Glu Trp Phe Phe Lys Asp Glu Lys Gly Asn Leu Leu
            100                 105                 110
Ser Arg Glu Ala Met Ile Glu Arg Met Arg Glu Tyr Ile His Thr Val
        115                 120                 125
Val Gly Arg Tyr Arg Gly Lys Val Tyr Ala Trp Asp Val Val Asn Glu
    130                 135                 140
Ala Val Asp Glu Asn Gln Pro Asp Gly Leu Arg Arg Ser Leu Trp Tyr
145                 150                 155                 160
Gln Val Ile Gly Pro Asp Tyr Ile Glu Leu Ala Phe Lys Phe Ala His
                165                 170                 175
Glu Ala Asp Pro Asp Ala Leu Leu Phe Tyr Asn Asp Tyr Asn Glu Phe
            180                 185                 190
Phe Pro Lys Lys Arg Asp Ile Ile Phe Lys Leu Val Lys Glu Met Arg
        195                 200                 205
Glu Lys Gly Val Pro Ile His Gly Ile Gly Met Gln Gln His Leu Thr
    210                 215                 220
Leu Ala Asp Asn Val Gly Trp Ile Asp Ile Ala Ile Gln Lys Phe Lys
225                 230                 235                 240
Thr Ile Ser Gly Ile Gln Ile His Ile Thr Glu Leu Asp Val Ser Val
                245                 250                 255
Tyr Lys Ser Arg Ser Pro Ser Ile Ile Tyr Gln Thr Pro Pro Leu Glu
            260                 265                 270
Val Leu Lys Glu Gln Ala Glu Phe Tyr Arg Lys Leu Phe Glu Ile Tyr
        275                 280                 285
Arg Lys His Thr Asp Val Ile Thr Asn Val Thr Phe Trp Gly Leu Lys
    290                 295                 300
Asp Asp Tyr Ser Trp Leu Arg Phe Phe Phe Gly Arg Arg Asn Asp Trp
305                 310                 315                 320
Pro Leu Leu Phe Asp Glu Asn Tyr Gln Pro Lys Pro Ala Phe Trp Ser
                325                 330                 335
Val Ile Glu Ser Val Ser Lys
            340

<210> 2
<211> 21
<212> PRT
<213> Pseudothermotoga thermarum

<400> 2
Met Lys Lys Leu Ile Phe Ala Ile Thr Leu Leu Leu Ile Val Phe Thr
1               5                   10                  15
Val Ile Ile Lys Gly
            20

<210> 3
<211> 364
<212> PRT
<213> Pseudothermotoga thermarum

<400> 3
Met Lys Lys Leu Ile Phe Ala Ile Thr Leu Leu Leu Ile Val Phe Thr
1               5                   10                  15
Val Ile Ile Lys Gly Gly Pro Gln Pro Met Ser Ser Gln Ile Pro Ser
            20                  25                  30
Leu Lys Asp Val Phe Leu Gln Asp Phe Lys Ile Gly Val Ala Leu Pro
        35                  40                  45
Val Arg Val Phe Ser Asn Ser Met Asp Val Glu Leu Ile Thr Lys His
    50                  55                  60
Phe Asn Ser Met Thr Ala Glu Asn Glu Met Lys Pro Glu Ser Ile Leu
65                  70                  75                  80
Arg Arg Asp Ala Ser Gly Lys Ile Tyr Tyr Asp Phe Thr Val Ala Asp
                85                  90                  95
Arg Tyr Ile Glu Phe Ala Gln Lys His Gly Met Val Val Arg Gly His
            100                 105                 110
Thr Leu Val Trp His Ser Gln Thr Pro Glu Trp Phe Phe Lys Asp Glu
        115                 120                 125
Lys Gly Asn Leu Leu Ser Arg Glu Ala Met Ile Glu Arg Met Arg Glu
    130                 135                 140
Tyr Ile His Thr Val Val Gly Arg Tyr Arg Gly Lys Val Tyr Ala Trp
145                 150                 155                 160
Asp Val Val Asn Glu Ala Val Asp Glu Asn Gln Pro Asp Gly Leu Arg
                165                 170                 175
Arg Ser Leu Trp Tyr Gln Val Ile Gly Pro Asp Tyr Ile Glu Leu Ala
            180                 185                 190
Phe Lys Phe Ala His Glu Ala Asp Pro Asp Ala Leu Leu Phe Tyr Asn
        195                 200                 205
Asp Tyr Asn Glu Phe Phe Pro Lys Lys Arg Asp Ile Ile Phe Lys Leu
    210                 215                 220
Val Lys Glu Met Arg Glu Lys Gly Val Pro Ile His Gly Ile Gly Met
225                 230                 235                 240
Gln Gln His Leu Thr Leu Ala Asp Asn Val Gly Trp Ile Asp Ile Ala
                245                 250                 255
Ile Gln Lys Phe Lys Thr Ile Ser Gly Ile Gln Ile His Ile Thr Glu
            260                 265                 270
Leu Asp Val Ser Val Tyr Lys Ser Arg Ser Pro Ser Ile Ile Tyr Gln
        275                 280                 285
Thr Pro Pro Leu Glu Val Leu Lys Glu Gln Ala Glu Phe Tyr Arg Lys
    290                 295                 300
Leu Phe Glu Ile Tyr Arg Lys His Thr Asp Val Ile Thr Asn Val Thr
305                 310                 315                 320
Phe Trp Gly Leu Lys Asp Asp Tyr Ser Trp Leu Arg Phe Phe Phe Gly
                325                 330                 335
Arg Arg Asn Asp Trp Pro Leu Leu Phe Asp Glu Asn Tyr Gln Pro Lys

340                 345                 350
Pro Ala Phe Trp Ser Val Ile Glu Ser Val Ser Lys
        355                 360

<210> 4
<211> 104
<212> PRT
<213> Artificial Sequence

<220>
<223> CBM2 domian

<400> 4
Thr Thr Thr Pro Val Pro Val Ser Gly Ser Leu Glu Val Lys Val Asn
1               5                   10                  15
Asp Trp Gly Ser Gly Ala Glu Tyr Asp Val Thr Leu Asn Leu Asp Gly
            20                  25                  30
Gln Tyr Asp Trp Thr Val Lys Val Lys Leu Ala Pro Gly Ala Thr Val
        35                  40                  45
Gly Ser Phe Trp Ser Ala Asn Lys Gln Glu Gly Asn Gly Tyr Val Ile
    50                  55                  60
Phe Thr Pro Val Ser Trp Asn Lys Gly Pro Thr Ala Thr Phe Gly Phe
65                  70                  75                  80
Ile Val Asn Gly Pro Gln Gly Asp Lys Val Glu Glu Ile Thr Leu Glu
                85                  90                  95
Ile Asn Gly Gln Val Ile Glu Ala
            100

<210> 5
<211> 191
<212> PRT
<213> Artificial Sequence

<220>
<223> CBM9 domain

<400> 5
Gly Ile Met Val Ala Thr Ala Lys Tyr Gly Thr Pro Val Ile Asp Gly
1               5                   10                  15
Glu Ile Asp Glu Ile Trp Asn Thr Thr Glu Glu Ile Glu Thr Lys Ala
            20                  25                  30
Val Ala Met Gly Ser Leu Asp Lys Asn Ala Thr Ala Lys Val Arg Val
        35                  40                  45
Leu Trp Asp Glu Asn Tyr Leu Tyr Val Leu Ala Ile Val Lys Asp Pro
    50                  55                  60
Val Leu Asn Lys Asp Asn Ser Asn Pro Trp Glu Gln Asp Ser Val Glu
65                  70                  75                  80
Ile Phe Ile Asp Glu Asn Asn His Lys Thr Gly Tyr Tyr Glu Asp Asp
                85                  90                  95
Asp Ala Gln Phe Arg Val Asn Tyr Met Asn Glu Gln Thr Phe Gly Thr
            100                 105                 110
Gly Gly Ser Pro Ala Arg Phe Lys Thr Ala Val Lys Leu Ile Glu Gly
        115                 120                 125
Gly Tyr Ile Val Glu Ala Ala Ile Lys Trp Lys Thr Ile Lys Pro Thr
    130                 135                 140
Pro Asn Thr Val Ile Gly Phe Asn Ile Gln Val Asn Asp Ala Asn Glu
145                 150                 155                 160
Lys Gly Gln Arg Val Gly Ile Ile Ser Trp Ser Asp Pro Thr Asn Asn
                165                 170                 175
Ser Trp Arg Asp Pro Ser Lys Phe Gly Asn Leu Arg Leu Ile Lys
            180                 185                 190

<210> 6
<211> 4
<212> PRT
<213> Artificial Sequence

<220>
<223> linker peptide

<400> 6
Val Asp Gly Ala
1

<210> 7
<211> 451
<212> PRT
<213> Artificial Sequence

<220>
<223> X11 with CBM2 domain

<400> 7
Gly Pro Gln Pro Met Ser Ser Gln Ile Pro Ser Leu Lys Asp Val Phe
1               5                   10                  15
Leu Gln Asp Phe Lys Ile Gly Val Ala Leu Pro Val Arg Val Phe Ser
            20                  25                  30
Asn Ser Met Asp Val Glu Leu Ile Thr Lys His Phe Asn Ser Met Thr
        35                  40                  45
Ala Glu Asn Glu Met Lys Pro Glu Ser Ile Leu Arg Arg Asp Ala Ser
    50                  55                  60
Gly Lys Ile Tyr Tyr Asp Phe Thr Val Ala Asp Arg Tyr Ile Glu Phe
65                  70                  75                  80
Ala Gln Lys His Gly Met Val Val Arg Gly His Thr Leu Val Trp His
                85                  90                  95
Ser Gln Thr Pro Glu Trp Phe Phe Lys Asp Glu Lys Gly Asn Leu Leu
            100                 105                 110
Ser Arg Glu Ala Met Ile Glu Arg Met Arg Glu Tyr Ile His Thr Val
        115                 120                 125
Val Gly Arg Tyr Arg Gly Lys Val Tyr Ala Trp Asp Val Val Asn Glu
    130                 135                 140
Ala Val Asp Glu Asn Gln Pro Asp Gly Leu Arg Arg Ser Leu Trp Tyr
145                 150                 155                 160
Gln Val Ile Gly Pro Asp Tyr Ile Glu Leu Ala Phe Lys Phe Ala His
                165                 170                 175
Glu Ala Asp Pro Asp Ala Leu Leu Phe Tyr Asn Asp Tyr Asn Glu Phe
            180                 185                 190
Phe Pro Lys Lys Arg Asp Ile Ile Phe Lys Leu Val Lys Glu Met Arg
        195                 200                 205
Glu Lys Gly Val Pro Ile His Gly Ile Gly Met Gln Gln His Leu Thr
    210                 215                 220
Leu Ala Asp Asn Val Gly Trp Ile Asp Ile Ala Ile Gln Lys Phe Lys
225                 230                 235                 240
Thr Ile Ser Gly Ile Gln Ile His Ile Thr Glu Leu Asp Val Ser Val
                245                 250                 255
Tyr Lys Ser Arg Ser Pro Ser Ile Ile Tyr Gln Thr Pro Pro Leu Glu
            260                 265                 270
Val Leu Lys Glu Gln Ala Glu Phe Tyr Arg Lys Leu Phe Glu Ile Tyr
        275                 280                 285
Arg Lys His Thr Asp Val Ile Thr Asn Val Thr Phe Trp Gly Leu Lys
    290                 295                 300
Asp Asp Tyr Ser Trp Leu Arg Phe Phe Phe Gly Arg Arg Asn Asp Trp

305                 310                 315                 320
Pro Leu Leu Phe Asp Glu Asn Tyr Gln Pro Lys Pro Ala Phe Trp Ser
                325                 330                 335
Val Ile Glu Ser Val Ser Lys Val Asp Gly Ala Thr Thr Thr Pro Val
            340                 345                 350
Pro Val Ser Gly Ser Leu Glu Val Lys Val Asn Asp Trp Gly Ser Gly
        355                 360                 365
Ala Glu Tyr Asp Val Thr Leu Asn Leu Asp Gly Gln Tyr Asp Trp Thr
    370                 375                 380
Val Lys Val Lys Leu Ala Pro Gly Ala Thr Val Gly Ser Phe Trp Ser
385                 390                 395                 400
Ala Asn Lys Gln Glu Gly Asn Gly Tyr Val Ile Phe Thr Pro Val Ser
                405                 410                 415
Trp Asn Lys Gly Pro Thr Ala Thr Phe Gly Phe Ile Val Asn Gly Pro
            420                 425                 430
Gln Gly Asp Lys Val Glu Glu Ile Thr Leu Glu Ile Asn Gly Gln Val
        435                 440                 445
Ile Glu Ala
    450

<210> 8
<211> 538
<212> PRT
<213> Artificial Sequence

<220>
<223> X11 with CBM9 domain

<400> 8
Gly Pro Gln Pro Met Ser Ser Gln Ile Pro Ser Leu Lys Asp Val Phe
1               5                   10                  15
Leu Gln Asp Phe Lys Ile Gly Val Ala Leu Pro Val Arg Val Phe Ser
            20                  25                  30
Asn Ser Met Asp Val Glu Leu Ile Thr Lys His Phe Asn Ser Met Thr
        35                  40                  45
Ala Glu Asn Glu Met Lys Pro Glu Ser Ile Leu Arg Arg Asp Ala Ser
    50                  55                  60
Gly Lys Ile Tyr Tyr Asp Phe Thr Val Ala Asp Arg Tyr Ile Glu Phe
65                  70                  75                  80
Ala Gln Lys His Gly Met Val Val Arg Gly His Thr Leu Val Trp His
                85                  90                  95
Ser Gln Thr Pro Glu Trp Phe Phe Lys Asp Glu Lys Gly Asn Leu Leu
            100                 105                 110
Ser Arg Glu Ala Met Ile Glu Arg Met Arg Glu Tyr Ile His Thr Val
        115                 120                 125
Val Gly Arg Tyr Arg Gly Lys Val Tyr Ala Trp Asp Val Val Asn Glu
    130                 135                 140
Ala Val Asp Glu Asn Gln Pro Asp Gly Leu Arg Arg Ser Leu Trp Tyr
145                 150                 155                 160
Gln Val Ile Gly Pro Asp Tyr Ile Glu Leu Ala Phe Lys Phe Ala His
                165                 170                 175
Glu Ala Asp Pro Asp Ala Leu Leu Phe Tyr Asn Asp Tyr Asn Glu Phe
            180                 185                 190
Phe Pro Lys Lys Arg Asp Ile Ile Phe Lys Leu Val Lys Glu Met Arg
        195                 200                 205
Glu Lys Gly Val Pro Ile His Gly Ile Gly Met Gln Gln His Leu Thr
    210                 215                 220
Leu Ala Asp Asn Val Gly Trp Ile Asp Ile Ala Ile Gln Lys Phe Lys
225                 230                 235                 240
Thr Ile Ser Gly Ile Gln Ile His Ile Thr Glu Leu Asp Val Ser Val
                245                 250                 255
Tyr Lys Ser Arg Ser Pro Ser Ile Ile Tyr Gln Thr Pro Pro Leu Glu

260                 265                 270
Val Leu Lys Glu Gln Ala Glu Phe Tyr Arg Lys Leu Phe Glu Ile Tyr
        275                 280                 285
Arg Lys His Thr Asp Val Ile Thr Asn Val Thr Phe Trp Gly Leu Lys
    290                 295                 300
Asp Asp Tyr Ser Trp Leu Arg Phe Phe Phe Gly Arg Arg Asn Asp Trp
305                 310                 315                 320
Pro Leu Leu Phe Asp Glu Asn Tyr Gln Pro Lys Pro Ala Phe Trp Ser
                325                 330                 335
Val Ile Glu Ser Val Ser Lys Val Asp Gly Ala Gly Ile Met Val Ala
            340                 345                 350
Thr Ala Lys Tyr Gly Thr Pro Val Ile Asp Gly Glu Ile Asp Glu Ile
        355                 360                 365
Trp Asn Thr Thr Glu Glu Ile Glu Thr Lys Ala Val Ala Met Gly Ser
    370                 375                 380
Leu Asp Lys Asn Ala Thr Ala Lys Val Arg Val Leu Trp Asp Glu Asn
385                 390                 395                 400
Tyr Leu Tyr Val Leu Ala Ile Val Lys Asp Pro Val Leu Asn Lys Asp
                405                 410                 415
Asn Ser Asn Pro Trp Glu Gln Asp Ser Val Glu Ile Phe Ile Asp Glu
            420                 425                 430
Asn Asn His Lys Thr Gly Tyr Tyr Glu Asp Asp Asp Ala Gln Phe Arg
        435                 440                 445
Val Asn Tyr Met Asn Glu Gln Thr Phe Gly Thr Gly Gly Ser Pro Ala
    450                 455                 460
Arg Phe Lys Thr Ala Val Lys Leu Ile Glu Gly Gly Tyr Ile Val Glu
465                 470                 475                 480
Ala Ala Ile Lys Trp Lys Thr Ile Lys Pro Thr Pro Asn Thr Val Ile
                485                 490                 495
Gly Phe Asn Ile Gln Val Asn Asp Ala Asn Glu Lys Gly Gln Arg Val
            500                 505                 510
Gly Ile Ile Ser Trp Ser Asp Pro Thr Asn Asn Ser Trp Arg Asp Pro
        515                 520                 525
Ser Lys Phe Gly Asn Leu Arg Leu Ile Lys
    530                 535

<210> 9
<211> 1032
<212> DNA
<213> Pseudothermotoga thermarum


<400> 9
ggaccacagc ccatgtcgtc acagatacca tcccttaagg atgttttttt gcaggatttc      60

aaaataggag tagctttacc agttagagtt ttcagcaatt cgatggatgt ggagttaatt     120

acgaagcact ttaacagtat gacggctgag aacgagatga aaccagagag tatattgaga     180

agggatgcga gtgggaagat ttactatgat tttacggttg cagacaggta catagagttt     240

gcgcagaaac atgggatggt tgttaggggt catacacttg tttggcacag tcagacgccg     300

gagtggtttt tcaaggatga gaagggcaat ttattgagca gggaagcgat gatagagagg     360

atgcgggagt acatacacac agttgtaggc aggtatagag gcaaggtgta tgcgtgggat     420

gtggtgaacg aggcggtgga tgagaatcag cctgatggtt tgaggaggag tttgtggtat     480

caggttatag ggccggatta catagagctt gccttcaaat ttgctcacga agcagatcca     540

gacgccttac tcttctacaa cgattacaac gaattttccc ccaaaaagcg agacatcatc     600

ttcaaattgg taaaagaaat gagagaaaag ggtgtaccga ttcatggtat tggcatgcag 660

caacatttaa ctcttgcaga taacgtaggt tggattgata ttgctattca aaaattcaag 720

accatttcag gtatacaaat tcacataact gagcttgatg ttagtgttta caaatcacgc 780

agtccatcta ttatctatca aacgccaccc ttggaggtgc tcaaggagca agcggaattt 840

taccgcaagt tgttcgaaat ctataggaaa cacactgacg tcataaccaa cgttaccttc 900

tggggtttga aagatgacta ttcgtggcta aggtttttct ttggtcgtag aaacgattgg 960

ccgcttttat ttgacgaaaa ttatcaaccc aaaccagctt tttggagtgt aatagaatca 1020

gtttccaagt aa 1032

<210> 10
<211> 63
<212> DNA
<213> Artificial Sequence

<220>
<223> signal peptide

<400> 10
atgaaaaagc tgattttcgc catcacattg ttgcttatag tctttactgt gataatcaaa 60

gga 63

<210> 11
<211> 12
<212> DNA
<213> Artificial Sequence

<220>
<223> Linker

<400> 11
gtcgacggcg ca 12

<210> 12
<211> 1029
<212> DNA
<213> Artificial Sequence

<220>
<223> X11 optimized

<400> 12
gggcctcagc cgatgtcgtc gcaaatcccc tcactgaagg atgtctttct tcaagatttc 60

aaaatcggcg tggcactgcc ggtacgcgtc ttctctaatt ccatggatgt tgagttaatt 120

accaaacatt tcaattcgat gacagcggaa aatgagatga agcccgaatc gattttacgt 180

cgtgatgcct cggggaagat ttattatgac ttcaccgttg ctgaccgcta catcgaattt 240

gcacagaagc atggcatggt ggtacgcgga cacacattag tttggcactc ccaaacaccg 300

gaatggtttt tcaaagatga aaagggcaat ttattgtcgc gtgaagcaat gattgaacgt 360

atgcgcgaat acatccacac agtagtaggc cgctaccgtg ggaaagttta cgcttgggac 420

gtcgttaatg aagccgtgga cgaaaaccag ccagacggac tgcgtcgttc gttatggtat 480

caggttatcg gcccagatta tattgagctt gcatttaagt ttgcgcatga ggctgaccca 540

gatgcattgt tattttacaa cgactataat gaattctttc cgaaaaaacg tgacatcatc 600

tttaagttgg ttaaggagat gcgcgaaaag ggcgtcccaa ttcatgggat cggaatgcaa 660

cagcatctta cacttgcgga caacgtgggg tggatcgata tcgcaatcca aaaatttaag 720

actatcagtg ggatccaaat ccatattacg gaacttgacg tatctgtcta caagagtcgt 780

tctccaagca ttatttatca gacacctcca cttgaagtgc ttaaagaaca agcggaattt 840

tatcgtaaac tttttgagat ttatcgcaag cacaccgacg taattaccaa tgttacattt 900

tgggggttga aggacgacta cagctggttg cgtttctttt ttggccgtcg caacgactgg 960

ccgctgcttt ttgacgagaa ttatcaaccg aaacctgcgt tttggagtgt tatcgaatca 1020

gtaagcaag 1029

<210> 13
<211> 1356
<212> DNA
<213> Artificial Sequence

<220>
<223> X11opt with CBM2 domain

<400> 13
gggcctcagc cgatgtcgtc gcaaatcccc tcactgaagg atgtctttct tcaagatttc 60

aaaatcggcg tggcactgcc ggtacgcgtc ttctctaatt ccatggatgt tgagttaatt 120

accaaacatt tcaattcgat gacagcggaa aatgagatga agcccgaatc gattttacgt 180

cgtgatgcct cggggaagat ttattatgac ttcaccgttg ctgaccgcta catcgaattt 240

gcacagaagc atggcatggt ggtacgcgga cacacattag tttggcactc ccaaacaccg 300

gaatggtttt tcaaagatga aaagggcaat ttattgtcgc gtgaagcaat gattgaacgt 360

atgcgcgaat acatccacac agtagtaggc cgctaccgtg ggaaagttta cgcttgggac 420

gtcgttaatg aagccgtgga cgaaaaccag ccagacggac tgcgtcgttc gttatggtat 480

caggttatcg gcccagatta tattgagctt gcatttaagt ttgcgcatga ggctgaccca 540

gatgcattgt tattttacaa cgactataat gaattctttc cgaaaaaacg tgacatcatc 600

tttaagttgg ttaaggagat gcgcgaaaag ggcgtcccaa ttcatgggat cggaatgcaa 660

cagcatctta cacttgcgga caacgtgggg tggatcgata tcgcaatcca aaaatttaag 720

actatcagtg ggatccaaat ccatattacg gaacttgacg tatctgtcta caagagtcgt 780

tctccaagca ttatttatca gacacctcca cttgaagtgc ttaaagaaca agcggaattt 840

tatcgtaaac tttttgagat ttatcgcaag cacaccgacg taattaccaa tgttacattt 900

tgggggttga aggacgacta cagctggttg cgtttctttt ttggccgtcg caacgactgg 960

ccgctgcttt ttgacgagaa ttatcaaccg aaacctgcgt tttggagtgt tatcgaatca 1020

gtaagcaagg tcgacggcgc aacaactacc cctgtcccag tctcaggatc tctagaggta 1080

aaggtaaacg attggggtag tggtgctgag tatgatgtga ctcttaattt ggatggacag 1140

tatgactgga ctgtgaaggt taaactagcc ccaggagcca ctgtaggaag cttctggagc 1200

gctaacaaac aagaggggaa tggctatgtc atcttcactc cagtaagctg gaataaaggg 1260

ccgacagcaa catttggatt catagtaaac ggaccacaag gagacaaagt agaagaaata 1320

accctagaaa taaacggaca agtaattgaa gcttaa 1356

<210> 14
<211> 1614
<212> DNA
<213> Artificial Sequence

<220>
<223> X11opt with CBM9 domain

<400> 14
gggcctcagc cgatgtcgtc gcaaatcccc tcactgaagg atgtctttct tcaagatttc 60

aaaatcggcg tggcactgcc ggtacgcgtc ttctctaatt ccatggatgt tgagttaatt 120

accaaacatt tcaattcgat gacagcggaa aatgagatga agcccgaatc gattttacgt 180

cgtgatgcct cggggaagat ttattatgac ttcaccgttg ctgaccgcta catcgaattt 240

gcacagaagc atggcatggt ggtacgcgga cacacattag tttggcactc ccaaacaccg 300

gaatggtttt tcaaagatga aaagggcaat ttattgtcgc gtgaagcaat gattgaacgt 360

atgcgcgaat acatccacac agtagtaggc cgctaccgtg ggaaagttta cgcttgggac 420

gtcgttaatg aagccgtgga cgaaaaccag ccagacggac tgcgtcgttc gttatggtat 480

caggttatcg gcccagatta tattgagctt gcatttaagt ttgcgcatga ggctgaccca 540

gatgcattgt tattttacaa cgactataat gaattctttc cgaaaaaacg tgacatcatc 600

tttaagttgg ttaaggagat gcgcgaaaag ggcgtcccaa ttcatgggat cggaatgcaa 660

cagcatctta cacttgcgga caacgtgggg tggatcgata tcgcaatcca aaaatttaag 720

actatcagtg ggatccaaat ccatattacg gaacttgacg tatctgtcta caagagtcgt 780

tctccaagca ttatttatca gacacctcca cttgaagtgc ttaaagaaca agcggaattt 840

tatcgtaaac tttttgagat ttatcgcaag cacaccgacg taattaccaa tgttacattt 900

tgggggttga aggacgacta cagctggttg cgtttctttt ttggccgtcg caacgactgg 960

ccgctgcttt ttgacgagaa ttatcaaccg aaacctgcgt tttggagtgt tatcgaatca 1020

gtaagcaagg tcgacggcgc aggaataatg gtagcgacag caaaatacgg aacaccggtc 1080

atcgatggag agatagacga gatctggaac acgacagagg agatagagac gaaagcggtg 1140

gccatgggat cgcttgacaa gaacgcgaca gcgaaagtga gggtgctgtg ggacgagaac 1200

tacctgtacg tacttgcaat cgtgaaagac cccgttctga acaaagacaa cagcaacccg 1260

tgggaacagg attccgtgga gatcttcatc gacgagaaca accacaagac aggatactac 1320

gaagacgacg acgcacagtt cagggtgaac tacatgaacg agcagacgtt tggaacggga 1380

ggaagtccag cgaggttcaa gacagcggtg aaactgatcg aaggaggata catagttgag 1440

gcagcgatca agtggaagac gatcaaaccc acaccgaaca cggtgatagg attcaacatc 1500

caggtgaacg atgcgaacga gaaagggcag agggtcggta tcatctcctg gagcgatccc 1560

acaaacaaca gctggagaga tccttcaaag ttcggtaacc tcaggctcat caag 1614

<210> 15
<211> 1095
<212> DNA
<213> Pseudothermotoga thermarum

<400> 15
atgaaaaagc tgattttcgc catcacattg ttgcttatag tctttactgt gataatcaaa 60

ggaggaccac agcccatgtc gtcacagata ccatccctta aggatgtttt tttgcaggat 120

ttcaaaatag gagtagcttt accagttaga gttttcagca attcgatgga tgtggagtta 180

attacgaagc actttaacag tatgacggct gagaacgaga tgaaaccaga gagtatattg 240

agaagggatg cgagtgggaa gatttactat gattttacgg ttgcagacag gtacatagag 300

tttgcgcaga aacatgggat ggttgttagg ggtcatacac ttgtttggca cagtcagacg 360

ccggagtggt ttttcaagga tgagaagggc aatttattga gcagggaagc gatgatagag 420

aggatgcggg agtacataca cacagttgta ggcaggtata gaggcaaggt gtatgcgtgg 480

gatgtggtga acgaggcggt ggatgagaat cagcctgatg gtttgaggag gagtttgtgg 540

tatcaggtta tagggccgga ttacatagag cttgccttca aatttgctca cgaagcagat 600

ccagacgcct tactcttcta caacgattac aacgaatttt tccccaaaaa gcgagacatc 660

atcttcaaat tggtaaaaga aatgagagaa aagggtgtac cgattcatgg tattggcatg 720

cagcaacatt taactcttgc agataacgta ggttggattg atattgctat tcaaaaattc 780

aagaccattt caggtataca aattcacata actgagcttg atgttagtgt ttacaaatca 840

cgcagtccat ctattatcta tcaaacgcca cccttggagg tgctcaagga gcaagcggaa 900

ttttaccgca agttgttcga aatctatagg aaacacactg acgtcataac caacgttacc 960

ttctggggtt tgaaagatga ctattcgtgg ctaaggtttt tctttggtcg tagaaacgat 1020

tggccgcttt tatttgacga aaattatcaa cccaaaccag ctttttggag tgtaatagaa 1080

tcagtttcca agtaa 1095

<210> 16
<211> 1092
<212> DNA
<213> Artificial Sequence

<220>
<223> X11 optimized

<400> 16
atgaaaaagc tgattttcgc catcacattg ttgcttatag tctttactgt gataatcaaa 60

ggagggcctc agccgatgtc gtcgcaaatc ccctcactga aggatgtctt tcttcaagat 120

ttcaaaatcg gcgtggcact gccggtacgc gtcttctcta attccatgga tgttgagtta 180

attaccaaac atttcaattc gatgacagcg gaaaatgaga tgaagcccga atcgatttta 240

cgtcgtgatg cctcggggaa gatttattat gacttcaccg ttgctgaccg ctacatcgaa 300

tttgcacaga agcatggcat ggtggtacgc ggacacacat tagtttggca ctcccaaaca 360

ccggaatggt ttttcaaaga tgaaaagggc aatttattgt cgcgtgaagc aatgattgaa 420

cgtatgcgcg aatacatcca cacagtagta ggccgctacc gtgggaaagt ttacgcttgg 480

gacgtcgtta atgaagccgt ggacgaaaac cagccagacg gactgcgtcg ttcgttatgg 540

tatcaggtta tcggcccaga ttatattgag cttgcattta agtttgcgca tgaggctgac 600

ccagatgcat tgttatttta caacgactat aatgaattct ttccgaaaaa acgtgacatc 660

atctttaagt tggttaagga gatgcgcgaa aagggcgtcc caattcatgg gatcggaatg 720

caacagcatc ttacacttgc ggacaacgtg gggtggatcg atatcgcaat ccaaaaattt 780

aagactatca gtgggatcca aatccatatt acggaacttg acgtatctgt ctacaagagt 840

cgttctccaa gcattattta tcagacacct ccacttgaag tgcttaaaga acaagcggaa 900

ttttatcgta aactttttga gatttatcgc aagcacaccg acgtaattac caatgttaca 960

ttttgggggt tgaaggacga ctacagctgg ttgcgtttct tttttggccg tcgcaacgac 1020

tggccgctgc tttttgacga gaattatcaa ccgaaacctg cgttttggag tgttatcgaa 1080

tcagtaagca ag 1092

<210> 17
<211> 1419
<212> DNA
<213> Artificial Sequence

<220>
<223> X11 optimized with SBM2

<400> 17
atgaaaaagc tgattttcgc catcacattg ttgcttatag tctttactgt gataatcaaa      60

ggagggcctc agccgatgtc gtcgcaaatc ccctcactga aggatgtctt tcttcaagat     120

ttcaaaatcg gcgtggcact gccggtacgc gtcttctcta attccatgga tgttgagtta     180

attaccaaac atttcaattc gatgacagcg gaaaatgaga tgaagcccga atcgatttta     240

cgtcgtgatg cctcggggaa gatttattat gacttcaccg ttgctgaccg ctacatcgaa     300

tttgcacaga agcatggcat ggtggtacgc ggacacacat tagtttggca ctcccaaaca     360

ccggaatggt ttttcaaaga tgaaaagggc aatttattgt cgcgtgaagc aatgattgaa     420

cgtatgcgcg aatacatcca cacagtagta ggccgctacc gtgggaaagt ttacgcttgg     480

gacgtcgtta atgaagccgt ggacgaaaac cagccagacg gactgcgtcg ttcgttatgg     540

tatcaggtta tcggcccaga ttatattgag cttgcattta agtttgcgca tgaggctgac     600

ccagatgcat tgttatttta caacgactat aatgaattct ttccgaaaaa acgtgacatc     660

atctttaagt tggttaagga gatgcgcgaa aagggcgtcc caattcatgg gatcggaatg     720

caacagcatc ttacacttgc ggacaacgtg gggtggatcg atatcgcaat ccaaaaattt     780

aagactatca gtgggatcca aatccatatt acggaacttg acgtatctgt ctacaagagt     840

cgttctccaa gcattattta tcagacacct ccacttgaag tgcttaaaga acaagcggaa     900

ttttatcgta aactttttga gatttatcgc aagcacaccg acgtaattac caatgttaca     960

ttttgggggt tgaaggacga ctacagctgg ttgcgtttct tttttggccg tcgcaacgac    1020

tggccgctgc tttttgacga gaattatcaa ccgaaacctg cgttttggag tgttatcgaa    1080

tcagtaagca aggtcgacgg cgcaacaact acccctgtcc cagtctcagg atctctagag    1140

gtaaaggtaa acgattgggg tagtggtgct gagtatgatg tgactcttaa tttggatgga    1200

cagtatgact ggactgtgaa ggttaaacta gccccaggag ccactgtagg aagcttctgg    1260

agcgctaaca aacaagaggg gaatggctat gtcatcttca ctccagtaag ctggaataaa    1320

gggccgacag caacatttgg attcatagta aacggaccac aaggagacaa agtagaagaa    1380

ataaccctag aaataaacgg acaagtaatt gaagcttaa                           1419

<210> 18
<211> 1677
<212> DNA
<213> Artificial Sequence

<220>
<223> X11 optimized with CBM9

<400> 18

atgaaaaagc tgattttcgc catcacattg ttgcttatag tctttactgt gataatcaaa 60

ggagggcctc agccgatgtc gtcgcaaatc ccctcactga aggatgtctt tcttcaagat 120

ttcaaaatcg gcgtggcact gccggtacgc gtcttctcta attccatgga tgttgagtta 180

attaccaaac atttcaattc gatgacagcg gaaaatgaga tgaagcccga atcgatttta 240

cgtcgtgatg cctcggggaa gatttattat gacttcaccg ttgctgaccg ctacatcgaa 300

tttgcacaga agcatggcat ggtggtacgc ggacacacat tagtttggca ctcccaaaca 360

ccggaatggt ttttcaaaga tgaaaagggc aatttattgt cgcgtgaagc aatgattgaa 420

cgtatgcgcg aatacatcca cacagtagta ggccgctacc gtgggaaagt ttacgcttgg 480

gacgtcgtta atgaagccgt ggacgaaaac cagccagacg gactgcgtcg ttcgttatgg 540

tatcaggtta tcggcccaga ttatattgag cttgcattta agtttgcgca tgaggctgac 600

ccagatgcat tgttatttta caacgactat aatgaattct ttccgaaaaa acgtgacatc 660

atctttaagt tggttaagga gatgcgcgaa aagggcgtcc caattcatgg gatcggaatg 720

caacagcatc ttacacttgc ggacaacgtg gggtggatcg atatcgcaat ccaaaaattt 780

aagactatca gtgggatcca aatccatatt acggaacttg acgtatctgt ctacaagagt 840

cgttctccaa gcattattta tcagacacct ccacttgaag tgcttaaaga acaagcggaa 900

ttttatcgta aactttttga gatttatcgc aagcacaccg acgtaattac caatgttaca 960

ttttgggggt tgaaggacga ctacagctgg ttgcgtttct tttttggccg tcgcaacgac 1020

tggccgctgc tttttgacga gaattatcaa ccgaaacctg cgttttggag tgttatcgaa 1080

tcagtaagca aggtcgacgg cgcaggaata atggtagcga cagcaaaata cggaacaccg 1140

gtcatcgatg gagagataga cgagatctgg aacacgacag aggagataga gacgaaagcg 1200

gtggccatgg gatcgcttga caagaacgcg acagcgaaag tgagggtgct gtgggacgag 1260

aactacctgt acgtacttgc aatcgtgaaa gaccccgttc tgaacaaaga caacagcaac 1320

ccgtgggaac aggattccgt ggagatcttc atcgacgaga acaaccacaa gacaggatac 1380

tacgaagacg acgacgcaca gttcagggtg aactacatga acgagcagac gtttggaacg 1440

ggaggaagtc cagcgaggtt caagacagcg gtgaaactga tcgaaggagg atacatagtt 1500

gaggcagcga tcaagtggaa gacgatcaaa cccacaccga acacggtgat aggattcaac 1560

atccaggtga acgatgcgaa cgagaaaggg cagaggtcgg gtatcatctc ctggagcgat 1620

cccacaaaca acagctggag agatccttca aagttcggta acctcaggct catcaag 1677

<210> 19
<211> 18
<212> DNA
<213> Artificial Sequence

<220>
<223> primer

<400> 19
cccgaaaagt gccacctg                                                    18

<210> 20
<211> 19
<212> DNA
<213> Artificial Sequence

<220>
<223> primer

<400> 20
gttctgaggt cattactgg                                                   19